# EUROPÄISCHE PATENTANMELDUNG

(11) **EP 1 430 879 A2**
(43) Veröffentlichungstag der Anmeldung: **23.06.2004**
(21) Anmeldenummer: 03028616.5
(22) Anmeldetag: 11.12.2003
(51) Int. Cl.: A61K 7/32

(54) **Arylsulfatase-Inhibitoren in Deodorantien und Antitranspiratien**

(30) Priorität: 20.12.2002 DE 10260954
(71) Anmelder: Henkel Kommanditgesellschaft auf Aktien, 40589 Düsseldorf-Holthausen (DE)
(72) Erfinder: Banowski, Bernhard, 40597 Düsseldorf (DE); Wadle, Armin, Dr., 40699 Erkrath (DE); Siegert, Petra, Dr., 42781 Haan (DE)

(57) **Zusammenfassung**

Gegenstand der Erfindung ist die Verwendung von ausgewählten Arylsulfatase-inhibierenden Substanzen in einer kosmetischen Deodorant- oder Antitranspirant-Zusammensetzung zur Verringerung des durch Zersetzung von Steroidestern verursachten Körpergeruchs.

## Beschreibung

Gegenstand der Erfindung ist die Verwendung von ausgewählten Arylsulfatase-inhibierenden Substanzen in einer kosmetischen Deodorant- oder Antitranspirant-Zusammensetzung zur Verringerung des durch die Zersetzung von Steroidestern verursachten Körpergeruchs.
Apokriner Schweiß stellt eine komplexe Mischung dar, die unter anderem Steroide, Cholesterin und andere Fette sowie ca. 10 % Eiweiße enthält. Die Zersetzungsprodukte des apokrinen Schweißes, die wesentlich zum Körpergeruch, insbesondere zum axillaren Körpergeruch, beitragen, lassen sich in zwei Klassen einteilen, zum einen kurz- und mittelkettige, insbesondere C₂-C₁₂-Fettsäuren, die linear, verzweigt, gesättigt oder ungesättigt sein können, zum anderen verschiedene Steroidhormone und deren Stoffwechselprodukte. So sind an dem typischen Körpergeruch, besonders an dem der Männer, die Stoffwechselprodukte der Androgene beteiligt, insbesondere Androstenol (5α-Androst-16-en-3β-ol, 5α-Androst-16-en-3α-ol) und Androstenon (5α-Androst-16-en-3-on).
Steroide selbst sind nicht wasserlöslich. Um mit den Körperflüssigkeiten abtransportiert werden zu können, liegen sie normalerweise als Sulfat oder als Glucuronid vor. Auf der Haut erfolgt die Spaltung dieser Steroidester in die flüchtigen freien Steroide durch hydrolytische Enzyme der Hautbakterien, insbesondere der coryneformen Bakterien. Prinzipiell sind dazu alle bakteriellen Exoesterasen in der Lage, besonders aber das Enzym Arylsulfatase.

Die erfindungsgemäß wirksamen Deodorant-Zusammensetzungen können an dieser Stelle eingreifen und die Tätigkeit der bakteriellen Exoesterasen hemmen. Damit unterscheiden sie sich von den rein bakteriostatischen oder bakteriziden Zusammensetzungen des Standes der Technik, die den Nachteil aufweisen können, die natürliche Mikroflora der Haut zu beeinträchtigen.

Die Bekämpfung von steroidal verursachtem Körpergeruch durch die Hemmung von Arylsulfatase ist im Stand der Technik bekannt, beispielsweise aus den Druckschriften US 5,643,559 und US 5,676,937. Diese Dokumente offenbaren allerdings nur eine geringe Anzahl Arylsulfatase-inhibierender Wirkstoffe.

Aufgabe der vorliegenden Erfindung war es, weitere Arylsulfatase-inhibierende Wirkstoffe zu identifizieren, um eine größere Variabilität, Flexibilität und Hautverträglichkeit bei der Formulierung kosmetischer Deodorantien zu ermöglichen. Die Identifizierung bekannter kosmetischer Wirkstoffe als Arylsulfatase-Inhibitoren ermöglicht darüber hinaus, die Dosierung dieser Wirkstoffe herabzusetzen. Die enzyminhibierende Wirkung zeigt sich häufig bereits bei niedrigen Wirkstoffkonzentrationen, bei denen noch keine bakteriostatische oder bakterizide Wirkung gefunden wird. Es hat sich überraschend herausgestellt, dass der Einsatz von Arylsulfatase-Inhibitoren in Deodorantien besonders bei Männern geeignet ist, die Entstehung von Körpergeruch zu verhindern. Dabei ist es dem Fachmann im Rahmen seiner allgemeinen Fachkenntnisse möglich, die Wirkstoffe in der Deodorant-Zusammensetzung hinsichtlich ihrer Menge und/oder ihrer Art geschlechtsspezifisch auf die jeweilige Anwendergruppe abzustimmen.

Gegenstand der vorliegenden Erfindung ist die Verwendung von mindestens einer Arylsulfatase-inhibierenden Substanz, ausgewählt aus Pflanzenextrakten, Riechstoffen, Flavonoiden, Isoflavonoiden, Polyphenolen und 6,7-disubstituierten 2,2-Dialkylchromanen oder -chromenen in einer kosmetischen Deodorant- oder Antitranspirant-Zusammensetzung zur Verringerung des durch die hydrolytische Zersetzung von Steroidestern verursachten Körpergeruchs.

In einer bevorzugten Ausführungsform sind die erfindungsgemäß verwendeten Pflanzenextrakte ausgewählt aus den Extrakten aus Meeresalgen, Apfelkernen, Pinienrinde (Pinus Pinaster), Trauben, Rotwein, Rosmarin, Hyssop (Ysop), Gewürznelke und Weidenröschen (Epilobium angustifolium, Willowherb).
Erfindungsgemäß werden die Pflanzenextrakte in Mengen von 0,001 bis 20 Gew.-%, bevorzugt 0,005 bis 10 und besonders bevorzugt 0,01 bis 5 Gew.-% Aktivsubstanz, jeweils bezogen auf die gesamte kosmetische Zusammensetzung, eingesetzt.

In einer weiteren bevorzugten Ausführungsform sind die erfindungsgemäß verwendeten Riechstoffe ausgewählt aus dem Rohstoff Protectate HR, einem Riechstoffgemisch erhältlich von Symrise, vormals Haarmann und Reimer. Protectate HR wird erfindungsgemäß als Arylsulfatase-Inhibitor in Mengen von 0,001 bis 5 Gew.-%, bevorzugt 0,01 bis 2 Gew.-% und besonders bevorzugt 0,1 bis 1 Gew.-%, jeweils bezogen auf die gesamte kosmetische Zusammensetzung, eingesetzt.

Die erfindungsgemäß als Arylsulfatase-Inhibitoren verwendeten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus α-Glucosylrutin, α-Glucosylmyricetin, α-Glucosylisoquercetin, α-Glucosylquercetin, Naringin (Aurantiin, Naringenin-7-rhamnoglucosid), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glycosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phlorizin und Neohesperidindihydrochalkon.
Erfindungsgemäß werden die Flavonoide in Mengen von 0,001 bis 1 Gew.-%, bevorzugt 0,005 bis 0,5 Gew.-% und besonders bevorzugt 0,01 bis 0,4 Gew.-%, jeweils bezogen auf die Flavonoidaktivsubstanz in der gesamten kosmetischen Zusammensetzung, eingesetzt.

In einer weiteren bevorzugten Ausführungsform sind die erfindungsgemäß verwendeten Arylsulfatase-Inhibitoren ausgewählt aus Isoflavonoiden, wobei hierzu die Isoflavone und die Isoflavon-Glycoside gezählt werden.
Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäßen Isoflavonen zählen beispielsweise Daidzein, Genistein, Prunetin, Biochanin, Orobol, Santal, Pratensein, Irigenin, Glycitein, Biochanin A und Formononetin. Als Isoflavone bevorzugt sind Daidzein, Genistein, Glycitein und Formononetin.

In den erfindungsgemäß verwendeten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Bevorzugte Beispiele für die erfindungsgemäß verwendeten Isoflavon-Glycoside sind Daidzin und Genistin.

Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind.
Erfindungsgemäß werden die Isoflavonoide in Mengen von 0,001 bis 1 Gew.-%, bevorzugt 0,005 bis 0,5 Gew.-% und besonders bevorzugt 0,01 bis 0,4 Gew.-%, jeweils bezogen auf die Isoflavonoid-Aktivsubstanz in der gesamten kosmetischen Zusammensetzung, eingesetzt.

In einer weiteren bevorzugten Ausführungsform sind die erfindungsgemäß verwendeten Arylsulfatase-Inhibitoren ausgewählt aus Polyphenolen. Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten - oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Pinienrinde, Eichenrinde sowie anderer Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic.
Erfindungsgemäß werden die Polyphenole in Mengen von 0,001 bis 10 Gew.-%, bevorzugt 0,005 bis 5 Gew.-% und besonders bevorzugt 0,08 bis 3 Gew.-%, jeweils bezogen auf die gesamte kosmetische Zusammensetzung, eingesetzt.

In einer weiteren bevorzugten Ausführungsform sind die erfindungsgemäß verwendeten Arylsulfatase-Inhibitoren ausgewählt aus 6,7-disubstituierten 2,2-Dialkylchromanen oder - chromenen der allgemeinen Formeln (I) oder (II), 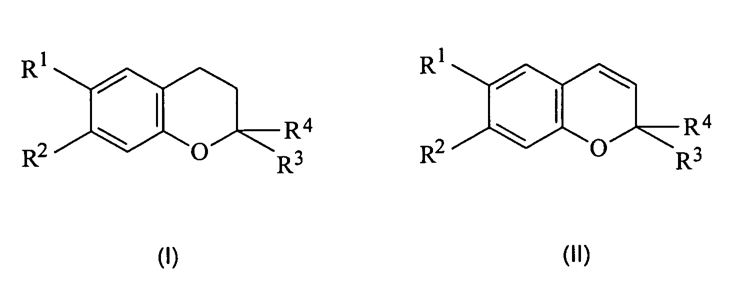 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen.

Die Substituenten R¹ und R² sind unabhängig voneinander ausgewählt aus einer OH-Gruppe, einer Methoxy-Gruppe und einer CF₃CH₂O-Gruppe. In einer bevorzugten Ausführungsform sind R¹ und R² unabhängig voneinander ausgewählt aus einer OH-Gruppe und einer Methoxy-Gruppe. In einer besonders bevorzugten Ausführungsform stellen R¹ eine OH-Gruppe und R² eine Methoxy-Gruppe dar.

Die Substituenten R³ und R⁴ stellen unabhängig voneinander eine C₁-C₄-Alkylgruppe dar. Unter C₁-C₄-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind R³ und R⁴ unabhängig voneinander ausgewählt aus einer Methyl-, Ethyl-, n-Propyl-, Isopropyl- und einer n-Butyl-Gruppe. Besonders bevorzugt stellen R³ und R⁴ unabhängig voneinander eine Methyl- oder Ethylgruppe dar. Außerordentlich bevorzugt ist, dass R³ und R⁴ identisch sind.

Erfindungsgemäß bevorzugt verwendet werden die 6,7-disubstituierten 2,2-Dialkylchromane. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCl-Bezeichnung Dimethylmethoxy Chromanol. Die Substanz ist unter dem Handelsnamen Lipochroman-6 von der Firma Lipotec S.A. erhältlich.

Die 6,7-disubstituierten 2,2-Dialkylchromane oder -chromene der allgemeinen Formeln (I) oder (II) werden erfindungsgemäß in Mengen von 0,001 - 5 Gew.-%, bevorzugt 0,005 - 2 Gew.-% und besonders bevorzugt 0,01 - 1 Gew.-%, jeweils bezogen auf die gesamte kosmetische Zusammensetzung, eingesetzt.

Ein weiterer Gegenstand der vorliegenden Erfindung ist ein Verfahren zur Verringerung von Körpergeruch mittels Inhibierung von Arylsulfatase auf der Haut, das dadurch gekennzeichnet ist, dass eine kosmetische Deodorant- oder Antitranspirant-Zusammensetzung, enthaltend mindestens eine Arylsulfatase-inhibierende Substanz, ausgewählt aus Pflanzenextrakten, Riechstoffen, Flavonoiden, Isoflavonoiden, Polyphenolen und 6,7-disubstituierten 2,2-Dialkylchromanen oder -chromenen, auf die Haut, insbesondere auf die Haut der Achselhöhlen, aufgetragen wird.

Die kosmetischen Deodorant- oder Antitranspirant-Zusammensetzungen, die die erfindungsgemäß verwendeten Arylsulfatase-Inhibitoren enthalten, können als Puder, in Stiftform, als Aerosolspray, Pumpspray, flüssige und gelförmige Roll on-Applikation, Creme, Gel und als getränktes flexibles Substrat vorliegen.
Deodorant- oder Antitranspirant-Stifte können in gelierter Form, auf wasserfreier Wachsbasis und auf Basis von W/O-Emulsionen und O/W-Emulsionen vorliegen. Gelstifte können auf der Basis von Fettsäureseifen, Dibenzylidensorbitol, N-Acylaminosäureamiden, 12-Hydroxystearinsäure und anderen Gelbildnern hergestellt werden.
Aerosolsprays, Pumpsprays, Roll on-Applikationen und Cremes können als Wasser-in-Öl-Emulsion, Öl-in-Wasser-Emulsion, Siliconöl-in-Wasser-Emulsion, Wasser-in-Öl-Mikroemulsion, Öl-in-Wasser-Mikroemulsion, Siliconöl-in-Wasser-Mikroemulsion, wasserfreie Suspension, alkoholische und hydroalkoholische Lösung, wässriges Gel und als Öl vorliegen. Alle genannten Zusammensetzungen können verdickt sein, beispielsweise auf der Basis von Fettsäureseifen, Dibenzylidensorbitol, N-Acylaminosäureamiden, 12-Hydroxystearinsäure, Polyacrylaten vom Carbomer- und Carbopol-Typ, Polyacrylamiden und Polysacchariden, die chemisch und/oder physikalisch modifiziert sein können.
Die Emulsionen und Mikroemulsionen können transparent, translucent oder opak sein.

Die kosmetischen Deodorant- oder Antitranspirant-Zusammensetzungen, die die erfindungsgemäß verwendeten Arylsulfatase-Inhibitoren enthalten, können weiterhin Fettstoffe enthalten. Unter Fettstoffen sind Fettsäuren, Fettalkohole, natürliche und synthetische kosmetische Ölkomponenten sowie natürliche und synthetische Wachse zu verstehen, die sowohl in fester Form als auch flüssig in wässriger oder öliger Dispersion vorliegen können.
Als Fettsäuren können eingesetzt werden lineare und/oder verzweigte, gesättigte und/oder ungesättigte C₈₋₃₀-Fettsäuren. Bevorzugt sind C₁₀₋₂₂-Fettsäuren. Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt ist der Einsatz von Stearinsäure. Die eingesetzten Fettsäuren können eine oder mehrere Hydroxygruppen tragen. Bevorzugte Beispiele hierfür sind die α-Hydroxy-C₈-C₁₈-Carbonsäuren sowie 12-Hydroxystearinsäure.
Die Einsatzmenge beträgt dabei 0,1 - 15 Gew.-%, bevorzugt 0,5 - 10 Gew.-%, besonders bevorzugt 1 - 5 Gew.-%, jeweils bezogen auf die gesamte Zusammensetzung.
Als Fettalkohole können eingesetzt werden gesättigte, ein- oder mehrfach ungesättigte, verzweigte oder unverzweigte Fettalkohole mit 6 - 30, bevorzugt 10 - 22 und ganz besonders bevorzugt 12 - 22 Kohlenstoffatomen. Einsetzbar im Sinne der Erfindung sind z.B. Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole.
Für Stiftformulierungen werden häufig Wachse verwendet. Als natürliche oder synthetische Wachse können erfindungsgemäß eingesetzt werden feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.
Weiterhin geeignet sind die Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs®) oder Polyolen mit 2 - 6 C-Atomen, Fettsäuremonoalkanolamide mit einem C₁₂₋₂₂-Acylrest und einem C₂₋₄-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/ oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen, sofern die einzelnen Wachskomponenten oder ihre Mischung bei Raumtemperatur fest sind.
Besonders bevorzugt ist, die Wachskomponenten zu wählen aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen, sofern die Wachskomponente oder die Gesamtheit der Wachskomponenten bei Raumtemperatur fest sind. Insbesondere vorteilhaft können die Wachskomponenten aus der Gruppe der C₁₆₋₃₆-Alkylstearate, der C₁₀₋₄₀-Alkylstearate, der C₂₋₄₀-Alkylisostearate, der C₂₀₋₄₀-Dialkylester von Dimersäuren, der C₁₈₋₃₈-Alkylhydroxystearoylstearate, der C₂₀₋₄₀-Alkylerucate gewählt werden, ferner sind C₃₀₋₅₀-Alkylbienenwachs sowie Cetearylbehenat einsetzbar. Auch Silikonwachse, zum Beispiel Stearyltrimethylsilan/Stearylalkohol sind gegebenenfalls vorteilhaft. Besonders bevorzugte Wachskomponenten sind die Ester aus gesättigten, einwertigen C₂₀-C₆₀-Alkoholen und gesättigten C₈-C₃₀-Monocarbonsäuren, insbesondere ein C₂₀-C₄₀-Alkylstearat bevorzugt, das unter dem Namen Kesterwachs® K82H von der Firma Koster Keunen Inc. erhältlich ist. Das Wachs oder die Wachskomponenten sollten bei 25° C fest sein, jedoch im Bereich von 35 - 95°C schmelzen, wobei ein Bereich von 45 - 85 °C bevorzugt ist.
Natürliche, chemisch modifizierte und synthetische Wachse können alleine oder in Kombination eingesetzt werden.
Die Wachskomponenten sind in einer Menge von 0,1 bis 40 Gew.-%, bezogen auf die Gesamtzusammensetzung, vorzugsweise 1 bis 30 Gew.-% und insbesondere 5 - 15 Gew.-% enthalten.
Die erfindungsgemäßen Zusammensetzungen können weiterhin wenigstens ein unpolares oder polares flüssiges Öl, das natürlich oder synthetisch sein kann, enthalten.
Die polare Ölkomponente kann ausgewählt sein aus pflanzlichen Ölen, z. B. Sonnenblumenöl, Olivenöl, Sojaöl, Rapsöl, Mandelöl, Jojobaöl und den flüssigen Anteilen des Kokosöls sowie synthetischen Triglyceridölen, aus Esterölen, das heißt den Estern von C₆-₃₀-Fettsäuren mit C₂₋₃₀―Fettalkoholen, aus Dicarbonsäureestern wie Di-n-butyladipat, Di-(2-ethylhexyl)-adipat und Di-(2-ethylhexyl)-succinat sowie Diolestern wie Ethylenglykoldioleat und Propylenglykoldi(2-ethylhexanoat), aus symmetrischen, unsymmetrischen oder cyclischen Estern der Kohlensäure mit Fettalkoholen, beispielsweise beschrieben in der DE-OS 197 56 454, Glycerincarbonat oder Dicaprylylcarbonat (Cetiol® CC), aus Mono,- Di- und Trifettsäureestern von gesättigten und/oder ungesättigten linearen und/oder verzweigten Fettsäuren mit Glycerin, z. B. Cutina® MD, aus verzweigten Alkanolen, z. B. Guerbet-Alkoholen mit einer einzigen Verzweigung am Kohlenstoffatom 2 wie 2-Hexyldecanol, 2-Octyldodecanol, Isotridecanol und Isohexadecanol, aus Alkandiolen, z. B. den aus Epoxyalkanen mit 12 - 24 C-Atomen durch Ringöffnung mit Wasser erhältlichen vicinalen Diolen, aus Etheralkoholen, z. B. den Monoalkylethern des Glycerins, des Ethylenglycols, des 1,2-Propylenglycols oder des 1,2-Butandiols, aus Dialkylethern mit jeweils 12 - 24 C-Atomen, z. B. den Alkyl-methylethern oder Di-n-alkylethern mit jeweils insgesamt 12 - 24 C-Atomen, insbesondere Di-n-octylether (Cetiol®OE ex Cognis), sowie aus Anlagerungsprodukten von Ethylenoxid und/oder Propylenoxid an ein- oder mehrwertige C₃₋₂₀-Alkanole wie Butanol und Glycerin, z. B. PPG-3-Myristylether (Witconol® APM), PPG-14-Butylether (Ucon Fluid® AP), PPG-15-Stearylether (Arlamol® E), PPG-9-Butylether (Breox® B25) und PPG-10-Butandiol (Macol® 57).
Die unpolare Ölkomponente kann ausgewählt sein aus flüssigen Paraffinölen, Isoparaffinölen, z. B. Isohexadecan und Isoeicosan, aus synthetischen Kohlenwasserstoffen, z. B. 1,3-Di-(2-ethyl-hexyl)-cyclohexan (Cetiol® S), sowie aus flüchtigen und nichtflüchtigen Siliconölen, die cyclisch, wie z. B. Decamethylcyclopentasiloxan und Dodecamethylcyclohexasiloxan, oder linear sein können, z. B. lineares Dimethylpolysiloxan, im Handel erhältlich z. B. unter der Bezeichnung Dow Corning® 190, 200, 244, 245, 344 oder 345 und Baysilon® 350 M.

Die erfindungsgemäßen Zusammensetzungen können weiterhin wenigstens einen wasserlöslichen Alkohol enthalten. Unter Wasserlöslichkeit versteht man erfindungsgemäß, dass sich wenigstens 5 Gew.-% des Alkohols bei 20 °C klar lösen oder aber ― im Falle langkettiger oder polymerer Alkohole - durch Erwärmen der Lösung auf 50 °C bis 60 °C in Lösung gebracht werden können. Geeignet sind je nach Darreichungsform einwertige Alkohole wie z. B. Ethanol, Propanol oder Isopropanol. Weiterhin geeignet sind wasserlösliche Polyole. Hierzu zählen wasserlösliche Diole, Triole und höherwertige Alkohole sowie Polyethylenglycole. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Hexandiole wie z. B. 1,6-Hexandiol. Weiterhin bevorzugt geeignet sind Glycerin und insbesondere Diglycerin und Triglycerin, 1,2,6-Hexantriol sowie die Polyethylenglycole (PEG) PEG-400, PEG-600, PEG-1000, PEG-1550, PEG-3000 und PEG-4000.
Die Menge des Alkohols oder des Alkohol-Gemisches in den erfindungsgemäßen Zusammensetzungen beträgt 1 - 50 Gew.-% und vorzugsweise 5 - 40 Gew.-%, bezogen auf die gesamte Zusammensetzung. Erfindungsgemäß kann sowohl ein Alkohol als auch ein Gemisch mehrerer Alkohole eingesetzt werden.

Die erfindungsgemäßen Zusammensetzungen können im wesentlichen wasserfrei sein, das heißt maximal 5 Gew.-%, bevorzugt maximal 1 Gew.-% Wasser enthalten. In wasserhaltigen Darreichungsformen beträgt der Wassergehalt 5 - 98 Gew.-%, bevorzugt 10 - 90 und besonders bevorzugt 15 - 85 Gew.-%, bezogen auf die gesamte Zusammensetzung.

Die erfindungsgemäßen Zusammensetzungen können weiterhin wenigstens ein hydrophil modifiziertes Silicon enthalten. Sie ermöglichen die Formulierung hochtransparenter Zusammensetzungen, reduzieren die Klebrigkeit und hinterlassen ein frisches Hautgefühl. Unter hydrophil modifizierten Siliconen werden erfindungsgemäß Polyorganosiloxane mit hydrophilen Substituenten verstanden, welche die Wasserlöslichkeit der Silicone bedingen. Erfindungsgemäß wird unter Wasserlöslichkeit verstanden, dass sich wenigstens 2 Gew.-% des mit hydrophilen Gruppen modifizierten Silicons in Wasser bei 20 °C lösen. Entsprechende hydrophile Substituenten sind beispielsweise Hydroxy-, Polyethylenglycol- oder Polyethylenglycol/Polypropylenglycol-Seitenketten sowie ethoxylierte Ester-Seitenketten. Erfindungsgemäß bevorzugt geeignet sind hydrophil modifizierte Silicon-Copolyole, insbesondere Dimethicone-Copolyole, die beispielsweise von Wacker-Chemie unter der Bezeichnung Belsil® DMC 6031, Belsil® DMC 6032, Belsil® DMC 6038 oder Belsil® DMC 3071 VP bzw. von Dow Corning unter der Bezeichnung DC 2501 im Handel sind. Besonders bevorzugt geeignet ist die Verwendung von Belsil® DMC 6038, da es die Formulierung hochtransparenter Zusammensetzungen ermöglicht, die beim Verbraucher eine höhere Akzeptanz erreichen. Erfindungsgemäß kann auch ein beliebiges Gemisch dieser Silicone eingesetzt werden.
Die Menge des hydrophil modifizierten Silicons oder des Alkohol-Gemisches in den erfindungsgemäßen Zusammensetzungen beträgt 0,5 - 10 Gew.-%, bevorzugt 1 - 8 Gew.-% und besonders bevorzugt 2 - 6 Gew.-%, bezogen auf das Gesamtgewicht der Zusammensetzung.

Die erfindungsgemäßen Zusammensetzungen können weiterhin wenigstens ein wasserlösliches Tensid enthalten. Als wasserlösliche Tenside eignen sich grundsätzlich alle in dem System zu 1 Gew.-% bei 20°C löslichen und in Wasser bei 20°C zu mindestens 1 Gew.-% löslichen Tenside. Obwohl die Struktur und lonogenität an sich unerheblich sind, scheinen nichtionische Tenside, insbesondere die bei Normaltemperatur (20°C) festen Anlagerungsprodukte des Ethylenoxids an Fettstoffmoleküle mit wenigstens einer alkoxylierbaren Gruppe, bevorzugt geeignet zu sein. Solche geeigneten Tenside sind z.B. die Anlagerungsprodukte von 10 - 40 Mol Ethylenoxid an lineare Fettalkohole mit 16 - 22 C-Atomen, an Fettsäuren mit 12 - 22 C-Atomen, an Fettsäurealkanolamide, an Fettsäuremonoglyceride, an Sorbitan-Fettsäuremonoester, an Fettsäurealkanolamide, an Fettsäureglyceride, z.B. an gehärtetes Rizinusöl, an Methylglucosidmonofettsäureester und Gemische davon.

Die erfindungsgemäßen Zusammensetzungen enthalten in einer bevorzugten Ausführungsform mindestens einen Antitranspirant-Wirkstoff. Als Antitranspirant-Wirkstoffe eignen sich wasserlösliche adstringierende metallische Salze, insbesondere anorganische und organische Salze des Aluminiums, Zirkoniums und Zinks bzw. beliebige Mischungen dieser Salze. Erfindungsgemäß wird unter Wasserlöslichkeit eine Löslichkeit von wenigstens 5 g Aktivsubstanz pro 100 g Lösung bei 20 °C verstanden. Erfindungsgemäß verwendbar sind beispielsweise Alaun (KAl(SO₄)₂ · 12 H₂O), Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-Zirkonium-Chlorohydrat, Zinkchlorid, Zinksulfocarbolat, Zinksulfat, Zirkoniumchlorohydrat und Aluminium-Zirkonium-Chlorohydrat-Glycin-Komplexe. Bevorzugt enthalten die Zusammensetzungen ein adstringierendes Aluminiumsalz, insbesondere Aluminiumchlorohydrat, und/oder eine Aluminium-Zirkonium-Verbindung. Die Antitranspirant-Wirkstoffe werden bei wässrigen Applikationen als wässrige Lösungen eingesetzt. In wasserfreien Zusammensetzungen werden die Antitranspirant-Wirkstoffe in fester Form eingesetzt. Sie sind in den erfindungsgemäßen Zusammensetzungen in einer Menge von 1 - 40 Gew.-%, vorzugsweise 5 - 30 Gew.-% und insbesondere 10 - 25 Gew.-% enthalten (bezogen auf die Menge der Aktivsubstanz in der Gesamtzusammensetzung). Aluminiumchlorohydrate werden beispielsweise pulverförmig als Micro Dry® Ultrafine von Reheis, als Chlorhydrol®, in aktivierter Form als Reach® 501 von Reheis sowie in Form einer wäßrigen Lösung als Locron® L von Clariant vertrieben. Unter der Bezeichnung Reach® 301 wird ein Aluminiumsesquichlorohydrat von Reheis angeboten. Auch die Verwendung von Aluminium-Zirkonium-Tetrachlorohydrex-Glycin-Komplexen, die beispielsweise von Reheis unter der Bezeichnung Rezal® 36G im Handel sind, ist erfindungsgemäß besonders vorteilhaft.

Weiterhin können die erfindungsgemäßen Zusammensetzungen keimhemmende oder desodorierende Wirkstoffe enthalten. Geeignet sind insbesondere Organohalogenverbindungen sowie -halogenide, quartäre Ammoniumverbindungen, eine Reihe von Pflanzenextrakten und Zinkverbindungen. Bevorzugt sind Chlorhexidin und Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar. Weitere antibakteriell wirksame Deodorans-Wirkstoffe sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide.
Flüssige und gelförmige Darreichungsformen können Verdickungsmittel enthalten, z. B. Celluloseether, wie Hydroxypropylcellulose, Hydroxyethylcellulose und Methylhydroxypropylcellulose, verdickende Polymere auf Basis von Polyacrylaten, die gewünschtenfalls vernetzt sein können, z. B. die Carbopoltypen oder Pemulen®-Produkte, oder auf Basis von Polyacrylamiden oder sulfonsäuregruppenhaltigen Polyacrylaten, z. B Sepigel® 305 oder Simulgel® EG, weiterhin anorganische Verdicker, z. B. Bentonite und Hectorite (Laponite®).
Die erfindungsgemäßen Zusammensetzungen können weitere kosmetisch und dermatologisch wirksame Stoffe enthalten, wie beispielsweise entzündungshemmende Substanzen, Feststoffe, ausgewählt aus Kieselsäuren, z. B. Aerosil®-Typen, Kieselgelen, Siliciumdioxid, Tonen, z. B. Bentonite oder Kaolin, Magnesiumaluminiumsilikaten, z. B. Talkum, Bornitrid, Titandioxid, das gewünschtenfalls beschichtet sein kann, gegebenenfalls modifizierten Stärken und Stärkederivaten, Cellulosepulvern und Polymerpulvern, desweiteren Pflanzenextrakte, Proteinhydrolysate, Vitamine, Parfümöle, Sebostatika, Anti-Akne-Wirkstoffe sowie Keratolytika.

Die kosmetischen Deodorant- oder Antitranspirant-Zusammensetzungen, die die erfindungsgemäß verwendeten Arylsulfatase-Inhibitoren enthalten, können, soweit sie flüssig vorliegen, auf flexible und saugfähige Träger aufgebracht und als Deodorant- oder Antitranspirant-Tücher oder Schwämmchen angeboten werden. Als flexible und saugfähige Träger im Sinne der Erfindung eignen sich z. B. Träger aus Textilfasern, Kollagen oder polymeren Schaumstoffen. Als Textilfasern können sowohl Naturfasern wie Cellulose (Baumwolle, Leinen), Seide, Wolle, Regeneratcellulose (Viskose, Rayon), Cellulosederivate als auch synthetische Fasern wie z.B. Polyester, Polyacrylnitril, Polyamid- oder Polyolefinfasern oder Mischungen solcher Fasern gewebt oder ungewebt verwendet werden. Diese Fasern können zu saugfähigen Wattepads, Vliesstoffen oder zu Geweben oder Gewirken verarbeitet sein. Auch flexible und saugfähige polymere Schaumstoffe, z. B. Polyurethanschäume und Polyamidschäume sind geeignete Substrate. Das Substrat kann eine, zwei, drei sowie mehr als drei Lagen aufweisen, wobei die einzelnen Lagen aus gleichen oder unterschiedlichen Materialien bestehen können. Jede Substratschicht kann eine homogene oder eine inhomogene Struktur mit beispielsweise verschiedenen Zonen unterschiedlicher Dichte aufweisen.

Als saugfähig im Sinne der Erfindung sind solche Trägersubstrate anzusehen, die bei 20° C wenigstens 10 Gew.-%, bezogen auf das Trockengewicht, an Wasser adsorptiv bzw. kapillar binden können. Bevorzugt eignen sich aber solche Träger, die wenigstens 100 Gew.-% Wasser adsorptiv und kapillar binden können.
Die Ausrüstung der Trägersubstrate erfolgt in der Weise, daß man die saugfähigen, flexiblen Trägersubstrate, bevorzugt aus Textilfasern, Kollagen oder polymeren Schaumstoffen mit den erfindungsgemäßen Zusammensetzungen behandelt bzw. ausrüstet und gegebenenfalls trocknet. Dabei kann die Behandlung (Ausrüstung) der Trägersubstrate nach beliebigen Verfahren, z. B. durch Aufsprühen, Tauchen und Abquetschen, Durchtränken oder einfach durch Einspritzen der erfindungsgemäßen Zusammensetzung in die Trägersubstrate erfolgen.

Erfindungsgemäß bevorzugt ist weiterhin die Darreichungsform als Aerosol, wobei die kosmetische Zusammensetzung ein Treibmittel, ausgewählt aus Propan, Butan, Isobutan, Pentan, Isopentan, Dimethylether, Fluorkohlenwasserstoffen und Fluorchlorkohlenwasserstoffen sowie Mischungen hiervon enthält.

Die folgenden Beispiele sollen die Erfindung verdeutlichen, ohne sie hierauf zu beschränken.

### Beispielrezepturen

| **Wasserfreie tensidhaltige AT-Stifte (Angaben in Gewichtsteilen)** | | | | | | | | | |
|---|---|---|---|---|---|---|---|---|---|
| | **1.1** | **1.2** | **1.3** | **1.4** | **1.5** | **1.6** | **1.7** | **1.8** | **1.9** |
| Eutanol® G 16 | 10 | - | - | 15 | 10 | - | 10 | - | 10 |
| Cetiol® OE | - | 10 | 15 | - | - | - | - | - | - |
| Ucon Fluid® AP | 5 | 5 | 5 | 5 | 5 | 5 | 5 | 5 | 5 |
| Cutina® HR | 6 | 6 | 6 | 6 | 6 | 6 | 2 | 5 | 6 |
| Lorol® C 18 | 20 | 20 | 20 | - | 20 | 20 | - | - | 20 |
| Lanette® O | - | - | - | 20 | - | - | 10 | 12 | - |
| Eumulgin® B 3 | 3 | 3 | 3 | 3 | 3 | 3 | 3 | 3 | - |
| Cutina® E 24 PF | - | - | - | - | 5 | - | - | - | - |
| Aluminiumchloro-hydrat | 20 | 20 | 20 | 20 | 20 | 20 | 20 | - | - |
| Talkum | 8 | 8 | 8 | 8 | 8 | 8 | 8 | 28 | 28 |
| Protectate HR | 0,1 | - | - | - | - | - | - | - | - |
| Ederline AAPP1-L - | | 0,5 | - | - | - | - | - | - | - |
| Emblica | - | - | 0,1 | - | - | - | - | - | - |
| Eurol BT | - | - | - | 0,1 | - | - | - | - | - |
| Lipochroman-6 | - | - | - | - | 0,05 - | | - | - | - |
| Herbalia® Olive, 12 Gew.-% wässrige Lösung | - | - | - | - | - | 0,2 | - | - | - |
| Pantrofina PC | - | - | - | - | - | - | 1,0 | - | - |
| Rosmarinextrakt 8%NC | - | - | - | - | - | - | - | 1,5 | - |
| Sepivinol R | - | - | - | - | - | - | - | - | 0,2 |
| Silikonöl DC® 245 | ad 100 | ad 100 | ad 100 | ad 100 | ad 100 | ad 100 | ad 100 | ad 100 | ad 100 |

| **Sprühfähige, translucente Antitranspirant-Mikroemulsionen (Angaben in Gew.-%)** | | | | | | | | |
|---|---|---|---|---|---|---|---|---|
| | **2.1** | **2.2** | **2.3** | **2.4** | **2.5** | **2.6** | **2.7** | **2.8** |
| Plantaren® 1200 | 1,71 | 1,71 | - | 1,71 | 1,71 | - | 1,71 | 1,71 |
| Plantaren® 2000 | 1,14 | 1,39 | 2,40 | 1,14 | 1,39 | 2,40 | 1,14 | 1,39 |
| Glycerinmonooleat | 0,71 | 0,71 | - | 0,71 | 0,71 | - | 0,71 | 0,71 |
| Dioctylether | 4,00 | 4,00 | 0,09 | 4,00 | 4,00 | 0,09 | 4,00 | 4,00 |
| Octyldodecanol | 1,00 | 1,00 | 0,02 | 1,00 | 1,00 | 0,02 | 1,00 | 1,00 |
| Parfümöl | 1,00 | 1,00 | 1,00 | 1,00 | 1,00 | 1,00 | 1,00 | 1,00 |
| Aluminiumchloro-hydrat | 8,00 | 5,00 | 5,00 - | | - | - | 8,00 | 5,00 |
| 1,2-Propylenglycol | 5,00 | 5,00 - | | 5,00 | 5,00 - | | 5,00 | 5,00 |
| Glycerin | - | - | 5,00 - | | - | 5,00 - | | - |
| Willowherb Extract | 1,0 | - | - | - | - | - | - | - |
| Meeresalgenextrakt - | | 0,6 | - | - | - | - | - | - |
| Herbasol MP Deo | - | - | 0,1 | - | - | - | - | - |
| Sepivinol R | - | - | - | 1,0 | - | - | - | - |
| Lipochroman-6 | - | - | - | - | 1,2 | - | - | - |
| Pantrofina PC | - | - | - | - | - | 1,7 | - | - |
| Rosmarinsäure | - | - | - | - | - | - | 0,01 | - |
| Ederline AAPP1-H | - | - | - | - | - | - | - | 1,0 |
| Wasser | ad 100 | ad 100 | ad 100 | ad 100 | ad 100 | ad 100 | ad 100 | ad 100 |

| **Seifenhaltige Stifte (Angaben in Gew.-%)** | | | | | | | | |
|---|---|---|---|---|---|---|---|---|
| | **3.1** | **3.2** | **3.3** | **3.4** | **3.5** | **3.6** | **3.7** | **3.8** |
| Ethanol | 22,5 | 22,5 | 22,5 | 22,5 | 22,5 | 22,5 | 22,5 | 22,5 |
| Cutina® FS 45 | 4,4 | 4,4 | 4,4 | 4,4 | 4,4 | 4,4 | 4,4 | 4,4 |
| 1,3 Butandiol | 31,7 | 31,7 | 31,7 | 31,7 | 31,7 | 31,7 | 31,7 | 31,7 |
| 1,2 Propylenglykol | 21,0 | 21,0 | 21,0 | 21,0 | 21,0 | 21,0 | 21,0 | 21,0 |
| Eutanol® G | 2,0 | 2,0 | 2,0 | 2,0 | 2,0 | 2,0 | 2,0 | 2,0 |
| Aethoxal® B | 3,0 | 3,0 | 3,0 | 3,0 | 3,0 | 3,0 | 3,0 | 3,0 |
| Cremophor® RH 455 | 0,05 | 0,05 | 0,05 | 0,05 | 0,05 | 0,05 | 0,05 | 0,05 |
| NaOH 45 %ig | 1,44 | 1,44 | 1,44 | 1,44 | 1,44 | 1,44 | 1,44 | 1,44 |
| Phenoxyethanol | 1,0 | 1,0 | 1,0 | 1,0 | 1,0 | 1,0 | 1,0 | 1,0 |
| Sensiva® SC 50 | 0,3 | 0,3 | 0,3 | 0,3 | 0,3 | 0,3 | 0,3 | 0,3 |
| Parfümöl | 2,0 | 2,0 | 2,0 | 2,0 | 2,0 | 2,0 | 2,0 | 2,0 |
| Ederline AAPP1-H | - | 1,0 | - | - | - | - | - | - |
| Herbalia® Olive, 12 Gew.-% wässrige Lösung | - | - | 0,2 | - | - | - | - | - |
| Lipochroman-6 | - | - | - | 0,7 | - | - | - | - |
| Ederline AAPP1-L | - | - | - | - | 0,015 - | | - | - |
| Sepivinol R | - | - | - | - | - | 0,05 | - | - |
| Pantrofina PC | - | - | - | - | - | - | 0,02 | - |
| Eurol BT | - | - | - | - | - | - | - | 1,0 |
| Wasser dest. | ad 100 | ad 100 | ad 100 | ad 100 | ad 100 | ad 100 | ad 100 | ad 100 |

| **Deodorant im Pumpzerstäuber (Angaben in Gew.-%)** | | | | | | |
|---|---|---|---|---|---|---|
| | **4.1** | **4.2** | **4.3** | **4.4** | **4.5** | **4.6** |
| Ethanol 96 %-ig, (DEP vergällt) | 55,0 | 55,0 | 55,0 | 55,0 | 55,0 | 55,0 |
| Triethylcitrat | 3,0 | 3,0 | 3,0 | 3,0 | 3,0 | 3,0 |
| Cremophor® RH 455 | 0,5 | 0,5 | 0,5 | 0,5 | 0,5 | 0,5 |
| Eurol BT | 1,0 | - | - | - | - | - |
| Pantrofina PC | - | 0,2 | - | - | - | - |
| Lipochroman-6 | - | - | 0,05 | - | - | - |
| Sepivinol R | - | - | - | 0,1 | - | - |
| Ederline AAPP1-H | - | - | - | - | 0,5 | - |
| Ederline AAPP1-L | - | - | - | - | - | 1,0 |
| Parfümöl | 1,0 | 1,0 | 1,0 | 1,0 | 1,0 | 1,0 |
| Wasser | ad 100 | ad 100 | ad 100 | ad 100 | ad 100 | ad 100 |

| **Wasserfreies Deodorant-Spray (Angaben in Gew.-%)** | | | |
|---|---|---|---|
| | **6.1** | **6.2** | **6.3** |
| 2-Octyldodecanol | 0,5 | 0,5 | 0,5 |
| Ethanol 99 %-ig, (DEP vergällt) | 39 | 39,45 | 38,95 |
| Protectate HR | 0,5 | - | 0,5 |
| Ederline AAPP1-L | - | 0,05 | 0,05 |
| n-Butan | 60 | 60 | 60 |

| **Antitranspirant Roll-on (Angaben in Gew.-%)** | | | | | |
|---|---|---|---|---|---|
| | **5.1** | **5.2** | **5.3** | **5.4** | **5.5** |
| Ethanol 96 %-ig,(DEP vergällt) | 30,0 | 30,0 | 30,0 | 30,0 | 30,0 |
| Mergital® CS 11 | 2,0 | 2,0 | 2,0 | 2,0 | 2,0 |
| Eumulgin® B 3 | 2,0 | 2,0 | 2,0 | 2,0 | 2,0 |
| Aluminiumchlorohydrat | 20,0 | 20,0 | 20,0 | 20,0 | 20,0 |
| Hydroxyethylcellulose | 0,5 | 0,5 | 0,5 | 0,5 | 0,5 |
| Ederline AAPP1-L | 0,01 | - | - | - | - |
| Eurol BT | - | 2,0 | - | - | - |
| Sepivinol R | - | - | 0,4 | - | - |
| Rosmarinextrakt 8% NC | | | | 0,7 | - |
| Lipochroman-6 | - | - | - | - | 0,015 |
| Parfümöl | 0,8 | 0,8 | 0,8 | 0,8 | 0,8 |
| Wasser | ad 100 | ad 100 | ad 100 | ad 100 | ad 100 |

### Antitranspirant-Tücher

Für die erfindungsgemäße Ausführungsform als Antitranspirant-Tuch wurde ein einlagiges Substrat aus 100 % Viskose mit einem Flächengewicht von 50 g/m² mit jeweils 75 g der Beispielemulsionen 2.1 bzw. 2.2 bzw. 2.3 pro Quadratmeter oder mit jeweils 75 g der Beispiellösungen 4.1 bzw. 4.2 beaufschlagt, in Tücher geeigneter Größe geschnitten und in Sachets verpackt.

| **Liste der eingesetzten Rohstoffe** | | |
|---|---|---|
| Rohstoff | **Beschreibung/INCl** | **Hersteller** |
| Aethoxal® B | PPG-5-Laureth-5 | Cognis |
| Cetiol® OE | Dicaprylyl Ether | Cognis |
| Cremophor® RH 455 | PEG-40 Hydrogenated Castor Oil, Aktivsubstanz 90 % | BASF |
| Cutina E 24 | PEG-20 Glyceryl Stearate | Cognis |
| Cutina® FS 45 | PALMITIC ACID, STEARIC ACID, Aktivsubstanz mind. 92 % | Cognis |
| Cutina® E 24 PF | PEG-20 GLYCERYL STEARATE | Cognis |
| Cutina® HR | Hydrogenated Castor Oil | Cognis |
| DC® 245 | Cyclopentasiloxane | Dow Corning |
| Ederline AAPP1-H | PEG 40-Hydrogenated Castor Oil, PPG-2 Ceteareth-9, Apple Extract | Seporga |
| Ederline AAPP1-L | Hexyl Decanol, Apple Extract | Seporga |
| Emblica | Phyllanthus emblica | Merck KGaA |
| Eumulgin® B 3 | Ceteareth-30 | Cognis |
| Eurol BT | Olive Leaf Extract, Water | B & T Srl, Milan |
| Eutanol® G 16 | Hexyldecyl Stearate | Cognis |
| Herbalia® Olive | Olea Europea (Olive) Leaf Extract (and) Maltodextrin (and) Silica | Cognis |
| Herbasol MP Deo | PEG-40 Hydrogenated Castor Oil, Hyssop Extract (Ysop), Clove Extract (Gewürznelke), Aqua | Cosmetochem |
| Lanette® O | Cetearyl Alcohol | Cognis |
| Lipochroman-6 | Dimethylmethoxy Chromanol | Merck KGaA |
| Lorol® C 18 | Stearyl Alcohol | Cognis |
| Mergital® CS 11 | Ceteareth-11 | Cognis |
| Oleanoline | Extrakt aus Olivenbaumblättern in Dipropylenglycol | Cognis |
| Pantrofina PC | Extrakt aus Pinienrinde (Pinus Pinaster) | Res Pharma Srl, Milan |
| Plantaren® 1200 | Lauryl Glucoside, ca. 50 % Aktivsubstanz | Cognis |
| Plantaren® 2000 | Decyl Glucoside, ca. 50 % Aktivsubstanz | Cognis |
| Protectate HR | Riechstoffgemisch | Symrise (Haarmann & Reimer) |
| Rosmarinextrakt 8% NC | Rosmarinextrakt wasserlöslich Pulver mit Standardmenge an 5% Rosmarinsäure, 3% Apigenin-7-glycosid | Dr. Marcus |
| Sepivinol R | Rotwein-Extrakt | Seppic |
| Ucon Fluid® AP | PPG-14 Butyl Ether | Amerchol (Union Carbide) |
| Weidenröschen-Extrakt | Willowherb Extract | Symrise (Dragoco) |

### Überprüfung der inhibitorischen Wirkung der Arylsulfatase-Inhibitoren (in vitro)

Da die hautrelevante Arylsulfatase nicht zur Verfügung stand, diente eine handelsübliche Arylsulfatase (EC 3.1.6.1) aus *Aerobacter aerogenes* als Modellenzym, um die Hemmwirkung der erfindungsgemäß verwendeten Inhibitoren zu testen. Die Tests wurden mit dem Sulfatase-Enzymassay Product No. S-1629 von Sigma gemäß den Angaben im *Sigma Quality Control Test Procedure*-Datenblatt durchgeführt. Als Referenz-Inhibitoren dienten Ascorbinsäure und ATP.

Zur Auswertung wurde die Arylsulfatase-katalysierte Bildung von p-Nitrophenol aus dem Substrat p-Nitrophenylsulfat (pNPS) spektrophotometrisch (λ = 420 nm) verfolgt.
Die Reaktionslösungen von 1000 µl, die auf eine Reaktionstemperatur von 37°C temperiert wurden, enthielten 187 mM Tris/HCl (pH 7,1), 8 mM pNPS und eine Startkonzentration von 0,05 U/ml Arylsulfatase. Die Einheit U der Enzymaktivität wurde so definiert, dass 1 U Sulfatase 1,0 µmol pNPS pro Minute bei pH 7,1 und 37°C hydrolysiert.
Die Testdurchführung ist auch offenbart bei H.R. Fowler und D. H. Rammler, Biochemistry 3, 230, 1964.

Die Reaktion wurde durch Zugabe des Enzyms gestartet und der Anstieg der Absorption bei λ = 420 nm über 5 Minuten verfolgt. Der lineare Anstieg der Absorption (A) pro Zeiteinheit (t) ist hierbei ein Maß für die Aktivität des Enzyms (ΔA/Δt). Die Aktivität des Enzyms in Abwesenheit eines Inhibitors, (ΔA₁/Δt₁ ), wurde als Referenz gleich 100% gesetzt. Unter analogen Bedingungen wurden die Aktivitäten in Gegenwart eines Inhibitors (ΔA₂/Δt₂) bestimmt. Die Hemmwirkung des Inhibitors bzw. die Minderung der Enzymaktivität wurde dann berechnet gemäß der Formel 100% - (ΔA₂/Δt₂ )/( ΔA₁/Δt₁ )%.

**Tabelle 1:**

| **Verschiedene Wirkstoffe als Inhibitor im Arylsulfatase-Enzymtest** | |
|---|---|
| **Rohstoff** | **Konzentration mit einer inhibierenden Wirkung von 50 %** [Gew.-% Rohstoff tel quel in wässriger Lösung] |
| Ederline AAPP1-H | 1,0 |
| Ederline AAPP1-L | 0,4 |
| Emblica | 0,025 |
| Eurol BT | 0,05 |
| Lipochroman-6 | 0,1 |
| Herbalia® Olive | 0,04 |
| Pantrofina PC | 0,4 |
| Rosmarinextrakt 8%NC | 0,005 |
| Sepivinol | 0,0025 |

## Patentansprüche

1. Verwendung von mindestens einer Arylsulfatase-inhibierenden Substanz, ausgewählt aus Pflanzenextrakten, Riechstoffen, Flavonoiden, Isoflavonoiden, Polyphenolen und 6,7-disubstituierten 2,2-Dialkylchromanen oder -chromenen, in einer kosmetischen Deodorant- oder Antitranspirant-Zusammensetzung zur Verringerung des durch die hydrolytische Zersetzung von Steroidestern verursachten Körpergeruchs.

2. Verwendung gemäß Anspruch 1, **dadurch gekennzeichnet, dass** die Pflanzenextrakte ausgewählt sind aus den Extrakten von Meeresalgen, Apfelkernen, Pinienrinde (Pinus Pinaster), Trauben, Rotwein, Rosmarin, Hyssop (Ysop), Gewürznelke und Weidenröschen (Epilobium angustifolium, Willowherb).

3. Verwendung gemäß Anspruch 1, **dadurch gekennzeichnet, dass** die Riechstoffe ausgewählt sind aus dem Rohstoff Protectate HR.

4. Verwendung gemäß Anspruch 1, **dadurch gekennzeichnet, dass** die Flavonoide ausgewählt sind aus Apigenin-7-glucosid, α-Glucosylrutin, α-Glucosylmyricetin, α-Glucosylisoquercetin, α-Glucosylquercetin, Naringin, Hesperidin, Neohesperidin, Rutin, Troxerutin, Monoxerutin, Diosmin, Eriodictin, Phlorizin und Neohesperidindihydrochalkon.

5. Verwendung gemäß Anspruch 1, **dadurch gekennzeichnet, dass** die Isoflavonoide ausgewählt sind aus Genistein, Daidzein, Glycitein, Formononetin, Genistin und Daidzin.

6. Verwendung gemäß Anspruch 1, **dadurch gekennzeichnet, dass** die Polyphenole ausgewählt sind aus Brenzcatechin, Resorcin, Hydrochinon, Phloroglucin, Pyrogallol, Hexahydroxybenzol, Anthocyanidinen, Flavonen, Gerbstoffen (Catechinen, Tanninen), Usninsäure, Acylpolyphenolen, den Derivaten der Gallussäure, den Derivaten der Digallussäure und der Digalloylgallussäure sowie den Polyphenolen aus Trauben und aus Rotwein.

7. Verwendung gemäß Anspruch 1, **dadurch gekennzeichnet, dass** die 6,7-disubstituierten 2,2-Dialkylchromane oder -chromene ausgewählt sind aus 3,4-Dihydro-7-methoxy-2,2-dimethyl-2H-1-benzopyran-6-ol.

8. Verfahren zur Verringerung von Körpergeruch mittels Inhibierung von Arylsulfatase auf der Haut, **dadurch gekennzeichnet, dass** eine kosmetische Deodorant- oder Antitranspirant-Zusammensetzung, enthaltend mindestens eine Arylsulfatase-inhibierende Substanz, ausgewählt aus Pflanzenextrakten, Riechstoffen, Flavonoiden, Isoflavonoiden, Polyphenolen und 6,7-disubstituierten 2,2-Dialkylchromanen oder - chromenen, auf die Haut, insbesondere auf die Haut der Achselhöhlen, aufgetragen wird.

9. Verfahren zur Verringerung von Körpergeruch gemäß Anspruch 8, **dadurch gekennzeichnet, dass** die Arylsulfatase-inhibierenden Substanzen hinsichtlich ihrer Menge und/oder ihrer Art geschlechtsspezifisch eingesetzt werden.

10. Verfahren zur Verringerung von Körpergeruch gemäß Anspruch 8, **dadurch gekennzeichnet, dass** die Arylsulfatase-inhibierenden Substanzen zur Verringerung von Körpergeruch beim Mann eingesetzt werden.
